# EUROPÄISCHE PATENTSCHRIFT

(11) **EP 1 440 669 B1**
(45) Veröffentlichungstag und Bekanntmachung des Hinweises auf die Patenterteilung: **22.11.2006**
(21) Anmeldenummer: 03001516.8
(22) Anmeldetag: 23.01.2003
(51) Int. Cl.: A61C 8/00, A61F 2/30

(54) **Knochenimplantat und Verfahren zu seiner Herstellung**
Bone implant and process for its manufacture
Implant osseux et procédé pour sa fabrication

(43) Veröffentlichungstag der Anmeldung: 28.07.2004
(73) Patentinhaber: Dinkelacker, Wolfgang, Dr. med. dent., 71063 Sindelfingen (DE)
(72) Erfinder: Dinkelacker, Wolfgang, Dr. med. dent., 71063 Sindelfingen (DE)
(74) Vertreter: Kindermann, Manfred

(56) Entgegenhaltungen:
- EP-A- 0 350 435
- EP-A- 0 800 802
- EP-A- 1 013 236
- WO-A-01/35871
- WO-A-86/02824
- WO-A-95/12369
- WO-A-96/16611
- WO-A-99/13700
- DE-A- 3 839 724
- DE-A- 4 211 343
- FR-A- 2 169 945
- FR-A- 2 610 512
- FR-A- 2 715 568
- US-A- 5 922 029
- US-A- 6 045 581

## Beschreibung

### Bereich der Erfindung

Die Erfindung bezieht sich auf ein Knochenimplantats mit mit einem vorzugsweise aus Titan oder einer Titanlegierung bestehenden Implantatkörper, der eine knochenverträgliche Oberfläche aufweist zur Erzielung einer sicheren Verankerung des Implantats im menschlichen Knochen, sowie auf ein verfahren zur Herstellung einer knochenverträglichen Oberfläche.

### Stand der Technik

Bekannte Knochenimplantate bestehen aus einem Material, das eine gute Verträglichkeit mit dem Gewebe des menschlichen Knochens aufweist. Besonders geeignet sind Titan und Titanlegierungen oder Tantal und Tantallegierungen. Um ein schnelles und dauerhaftes Einwachsen des Implantats im Knochen zu fördern, werden die bekannten Implantate in den Bereichen ihres Kontaktes mit dem Knochen mit einer Oberflächenrauhigkeit versehen, durch die eine Anlagerung der Knochenzellen an der Implantatoberfläche verbessert und der Widerstand des eingewachsenen Implantats gegen seitliche Kräfte vergrößert wird. Die Art der Oberflächenrauhigkeit und ihre Abmessungen sind von entscheidender Bedeutung für eine sichere und dauerhafte Verankerung des Implantats im Knochen. Man unterscheidet dabei zwischen einer Makrorauhigkeit in Form von schrauben-, rippen- nuten-, rillen- oder gitterartigen Strukturen und einer Mikrorauhigkeit in Form von Poren, Mulden oder Lakunen, die gegenüber den Strukturen der Makrorauhigkeit klein sind (EP-A 1013236). Im Bereich der Makrorauhigkeit liegen die Rauhigkeitswerte zwischen 10-150µm und im Bereich der Mikrorauhigkeit zwischen 0,5-3µm.

Um eine Implantatoberfläche mit biokompatiblen Eigenschaften zu erreichen, wurden umfangreiche Untersuchungen durchgeführt (z.B. Buser, D. et al, "Influence of Surface Characteristics on Bone Integration of Titanium Implants", Journal of Biomedical Materials Research, Vol. 25, S. 889-902, John Wiley & Sons, Inc., 1991). Durch eine Behandlung der Oberfläche des Implantatkörpers soll der Kontakt mit dem umgebenden Knochengewebe erleichtert und die daran beteiligte Implantatoberfläche vergrößert werden. Es ist auch bekannt, die Oberfläche des Implantatkörpers nach einer Entfernung der natürlichen Titanoxydschicht einem Säureätzprozess auszusetzen, um eine im wesentlichen gleichförmige Rauhigkeit über die ganze Oberfläche zu erzielen (WO 96/16611). Des weiteren sind Oberflächenbeschichtungen mit Materialien bekannt, die eine gute Knochenverträglichkeit aufweisen wie z.B. eine Beschichtung mit Hydroxylapatit (DE-A 38 39 724) oder mit Perlmutpulver (FR-A 2715568).

Es sind ferner Titan-Plasmabeschichtungen (TPS) bekannt, die durch Thermospritzen von Titan auf ein Titanimplantat erzeugt werden. Ein weiteres bekanntes Verfahren sieht vor, dass zunächst eine Behandlung der Oberfläche durch Grobsandstrahlen mit Korund erfolgt, wodurch eine Makrorauheit auf dem Titan erzielt wird. Diesem Vorgang folgt eine Säureätzung, durch die über die sandgestrahlten Oberfläche gleichförmig verteilte Mikrogrübchen erzeugt werden (Cochran et al, "Bone response to unloaded and loaded titanium implants with a sand blasted and acid-etched surface", Journal of Biomedical Materials Research, Vol. 40, 1998, S. 1; EP-A 0388567).

Es ist auch bekannt, die Oberfläche von Metallimplantaten durch Ionenstrahl-Sputtern zu behandeln, um ihre Biokompatibilität zu verbessern (Weigand, A.J. et al, "Ion-beam-sputter modification of the surface morphology of biological implants", Journal Vacuum Society Technology, Vol. 14, No.1, Jan./Feb. 1977, S. 326-331).

Durch US-A-5,922,029 (Wagner et al.) ist es bekannt, bei einer durch Ätzen behandelten Implantatoberfläche die Unregelmäßigkeit in Form und Verteilung der Erhebungen und Vertiefungen, die durch das Ätzen erzeugt werden, gezielt dadurch zu erhöhen, dass vor dem Ätzen bestimmte Punkte der Oberfläche durch Auftragen einer Maskierschicht maskiert werden. Erst danach findet der Ätzvorgang statt, bei dem die maskierten Bereiche vor einem Angreifen des Ätzmittels geschützt bleiben. Diese Schritte werden mehrfach wiederholt. Dieses Verfahren beruht auf der Erkenntnis, dass eine unregelmäßige Oberflächenstruktur das Einwachsen des Implantats im Knochen fördert.

Es sind auch zahnimplantate bekannt, die an ihrer Oberfläche rillenförmige Vertiefungen aufweisen. Zusätzlich zu diesen Vertiefungen sind Mulden oder Lagunen vorgesehen, die gegenüber den rillenförmigen Vertiefungen klein sind und sich mit hoher Dichte über die Oberfläche der rillenförmige Vertiefungen verteilen. Die Mulden oder Lagunen werden durch Ätzen hergestellt (EP-A-1 013 236).

Bei einem Teil der bekannten Verfahren kann die mangelnde Härte und Haftfähigkeit der Oberflächenbeschichtung ein Problem darstellen. Bei anderen Verfahren werden Verunreinigungen der Implantatoberfläche erzeugt, die durch aufwendige Verfahrensschritte wieder entfernt werden müssen. Ein Beispiel hierfür ist die Behandlung der Implantatoberfläche durch Sandstrahlen, das Einschlüsse von Strahlgutpartikeln auf der Implantatoberfläche hinterlässt. Diese Einschlüsse können das Einwachsen des Implantats erschweren und müssen daher durch anschließendes Säureätzen entfernt werden. Von besonderem Nachteil es, dass das Sandstrahlen und die Behandlung der Implantatoberfläche mit Säure eine scharfkantige Oberflächenstruktur hinterläßt, die spitze Zacken und Dornen und spitz auslaufende Vertiefungen aufweist. Die Biokompatibilität einer scharfkantig zerklüfteten Oberfläche ist wegen unzureichender Anpassung an die Form der sich am Implantat anlagernden Knochenzellen und deren Zellenfortsätzen eingeschränkt.

### Zusammenfassung der Erfindung

Ziel der Erfindung ist es, unter Vermeidung der genannten Nachteile eine Oberfläche von Knochenimplantaten herzustellen, die eine verbesserte Biokompatibilität bei ausreichender Härte und Haftfähigkeit aufweist.

Die Erfindung findet vorzugsweise bei einem Knochenimplantat Anwendung, das aus Titan oder einer Titanlegierung besteht und in den Teilen seiner Oberfläche, die mit dem Knochen in Kontakt kommen, knochenverträglich ausgestaltet ist. Zu diesem Zweck haben diese Oberflächenteile eine Makrostruktur zur Anlagerung von Knochensubstanz sowie eine der Makrostruktur überlagerte Mikrostruktur zur Verankerung des Implantats in Zellebene. Nach der Erfindung, wie sie in den Ansprüchen definiert ist, ist über einer durch Sandstrahlen und/oder Säureätzen vorbehandelten Oberfläche des Implantatkörpers eine aus Titan oder einer Titanlegierung bestehende Deckschicht vorgesehen, die vom Sandstrahlen und/oder Säureätzen erzeugte Spitzen und schartenartige Vertiefungen zu einer eine Vielzahl gerundeter Erhöhungen (Kuppen) und gerundeter Lakunen aufweisenden Mikrostruktur glättet, wobei die Größe der Erhöhungen und die Tiefe der Lakunen der Größenordnung der Knochenzellen angepasst ist.

Ein solches Implantat hat den Vorzug, dass die Grenzfläche, an der sich das Knochengewebe anlagert, einer Topographie besitzt, die weitgehend der Form der Knochenzellen entspricht, die ausschließlich gerundete und kantenlose Strukturen aufweisen. Hierdurch wird die Anpassung der Zellen an die Grenzfläche und ihre Migration bei Belastung des Implantats erleichtert.

Nach einer bevorzugten Ausführungsform ist die Deckschicht eine durch Sputtern aufgebrachte Schicht, deren Dicke wenigstens annähernd der Dicke der Knochenzellen (Osteozyten) entspricht. In vorteilhafter Weise weist die Deckschicht eine Dicke zwischen 0,1 und 2 Mikrometern auf.

Gegenstand der in den Ansprüchen definierten Erfindung ist des weiteren ein Verfahren zur Herstellung eines Knochenimplantats, insbesondere eines zahnimplantats, das einen vorzugsweise aus Titan oder einer Titanlegierung bestehenden Implantatkörper mit einer biokompatiblen Oberfläche aufweist, die eine Makrostruktur zur Anlagerung von Knochensubstanz und eine Mikrostruktur zur Verankerung des Implantats im Zellbereich besitzt.
Nach der Erfindung, wie sie in den Ansprüchen definiert ist, wird diese Oberfläche durch eine Vorbehandlung mittels Sandstrahlen und/oder Säureätzen aufgerauht und auf die vorbehandelte Oberfläche des Implantatkörpers wird eine aus Titan oder einer Titanlegierung bestehende Deckschicht aufgebracht, durch die vom Sandstrahlen und/oder Säureätzen der Vorbehandlung erzeugte Spitzen und schartenartige Vertiefungen zu einer eine Vielzahl gerundeter Erhöhungen (Kuppen) und gerundeter Lakunen aufweisenden Mikrostruktur umgeformt werden, wobei die Größe der Erhöhungen und die Tiefe der Lakunen der Größenordnung der Knochenzellen angepasst ist. Die Deckschicht wird vorzugsweise durch Sputtern aufgebracht.
Ein Vorteil des erfindungsgemäßen Verfahrens besteht darin, dass Verunreinigungen, die sich während des Sandstrahlens in die Oberfläche des Implantats einlagern, von der Deckschicht bleibend eingeschlossen werden. Die durch Sputtern aufgebrachte Deckschicht weist eine hohe Härte und eine gute Haftung auf der Implantatoberfläche auf. Die durch Sandstrahlen und/oder Säureätzen entstehenden Zacken, Spitzen und scharfkantige Einschnitte in der Mikrostruktur der Implantatoberfläche werden durch die Deckschicht abgerundet und ausgeglichen. Zellschädigungen bei der Anlagerung der Knochenzellen an die Implantatoberfläche werden so vermieden.

Nach einer weiteren vorteilhaften Ausführungsform der Erfindung wird der aus abgerundeten Erhöhungen (Kuppen) und zwischen diesen befindliche gerundeten Lakunen gebildete Mikrostruktur eine gleichartige Nanostruktur überlagert, deren Erhöhungen, Abstände und Lakunentiefe annähernd um eine dezimale Größenordnung kleiner ist als die der Mikrostruktur. Die Nanostruktur bietet Angriffspunkte für die Zellfortsätze und erleichtert damit die Migration der Knochenzellen.

### Beschreibung der Zeichnungen

Nachfolgend sind Ausführungsbeispiele der Erfindung anhand von Zeichnungen beschrieben. Es zeigen:
- Figur 1: eine stark vergrößerte Schnittdarstellung eines Teils der Oberfläche eines Knochenimplantats nach dem Stand der Technik;
- Figur 2: eine schematische Darstellung von zwei Knochenzellen in Anlagerung an einen Teil einer bekannten Implantatoberfläche;
- Figur 3: eine stark vergrößerten Teilschnitt eines ersten Ausführungsbeispiels einer erfindungsgemäß umgeformten Oberfläche des Knochenimplantats gemäß Figur 1;
- Figur 4: eine vergrößerte schematische Darstellung von mehreren Knochenzellen in Anlagerung an einen Teil einer Implantatoberfläche gemäß der Erfindung;
- Figur 5: eine stark vergrößerte Schnittdarstellung eines Teils eines weiteren Ausführungsbeispiels des erfindungsgemäß hergestellten Knochenimplantats mit einer vorbehandelten Oberfläche und einer Deckschicht;
- Figur 6: eine schematisierte Ansicht einer durch Sputtern hergestellten Deckschicht in räumlicher Darstellung;
- Figur 7: eine vergrößerte Schnittdarstellung eines Teils einer Implantatoberfläche, die eine Makrostruktur in Form von rillenförmigen Vertiefungen und eine die Makrostruktur überdeckende Mikrostruktur gemäß der Erfindung aufweist; und
- Figur 8: eine vergrößerte Schnittdarstellung eines Teils einer Implantatoberfläche, die eine erfindungsgemäße Mikrostruktur in und eine dieser überlagerte Nanostruktur aufweist.

### Detaillierte Beschreibung der dargestellten Ausführungsbeispiele

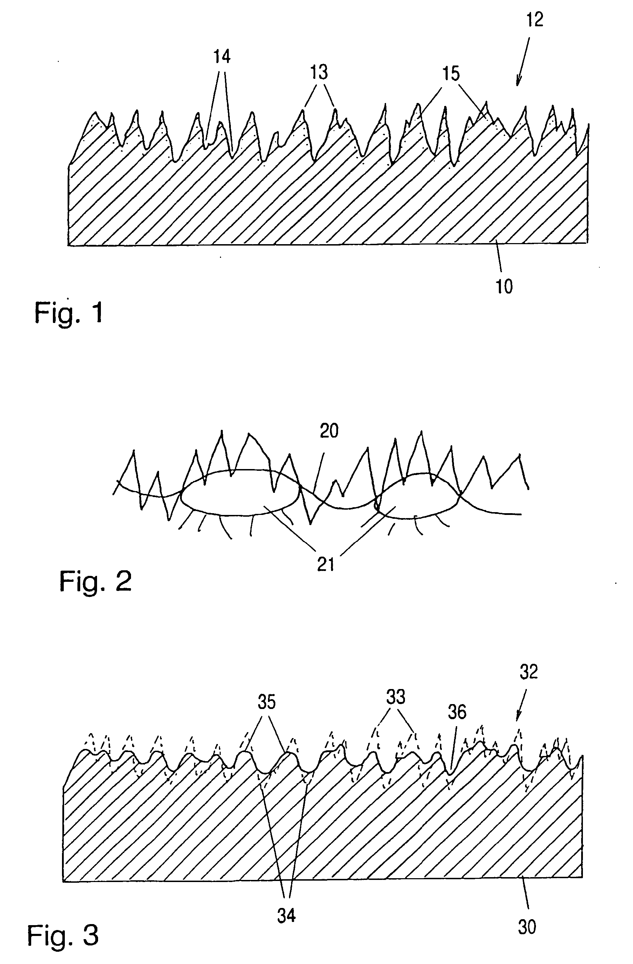

Die Schnittdarstellung von Figur 1 zeigt den Ausschnitt der Oberfläche eines bekannten Knochenimplantats 10, dessen Oberfläche 12 in bekannter Weise durch Sandstrahlen mit Korund oder Aluminiumoxyd behandelt wurde. Hierdurch wird eine Mikrostruktur erzeugt, die aus einer Vielzahl von Zacken und Spitzen 13 und zwischen diesen befindlichen scharfkantigen schartenartigen Vertiefungen 14 besteht. Diese Struktur stellt eine mangelhafte Anpassung an die Struktur der Knochensubstanz im Zellbereich dar (Figur 2). Bei der Anlagerung von Zellen 21 am Implantat 20 können Zellschädigungen auftreten, die ein Einwachsen des Implantats im Knochen verzögern oder auch zum Verlust des Implantats führen können. Außerdem können sich in der Oberfläche 12 Partikel 15 des Strahlguts zurückbleiben, die verunreinigungen des knochenkompatiblen Implantatmaterials darstellen und lokale Entzündungen hervorrufen können, die den Einheilprozess des Implantats beeinträchtigen.

Die Schnittdarstellung von Figur 3 zeigt einen entsprechenden Ausschnitt der Oberfläche eines Knochenimplantats 30, bei der die Mikrostruktur erfindungsgemäß umgeformt wurde. Bei dem Knochenimplantat 30 kann es sich um ein Zahnimplantat oder um ein Implantat zum Ersatz eines Hüftgelenks oder eines Kniegelenks oder um ein anderes zum Einsetzen in den menschlichen Körper geeignetes Knochenimplantat handeln. Das Implantat 30 besteht aus einem Material, das mit der Knochensubstanz des menschlichen Körpers gut verträglich ist und auch die erforderliche Härte aufweist. Vorzugsweise sind dies Titan und Titanlegierungen. Stattdessen können in bestimmten Fällen auch Tantal oder Tantallegierungen oder Keramische Werkstoffe zum Einsatz kommen. Die Oberfläche 32 wird durch Sandstrahlen mit Korund oder Aluminiumoxyd vorbehandelt, so dass eine Oberflächenstuktur mit Vielzahl von scharfkantigen Zacken und Spitzen 33 und zwischen diesen befindlichen scharfkantigen, schartenartigen Vertiefungen 34 erzeugt wird, die der gezackten Oberflächenstruktur 13, 14 von Figur 1 entspricht. In einem weiteren Verfahrensschritt wird die durch die Vorbehandlung aufgerauhte Oberfläche durch Materialauf- oder -abtragung so umgeformt, dass die Zacken und Spitzen 33 abgetragen und die schartenartigen Vertiefungen 34 teilweise ausgefüllt werden. Ergebnis der Umformung ist eine Vielzahl dicht benachbarter abgerundeter Erhöhungen oder Kuppen 35 und zwischen diesen befindliche gerundete Lakunen 36 mit einer mittleren Rauhtiefe im Bereich von 1-10µm, vorzugsweise von 1,5µm, und einem Durchmesser im Bereich von 1,5-10µm, vorzugsweise von 5µm.

Die Abrundung der Zacken und Spitzen 33 erfolgt vorzugsweise durch Einwirkung eines leistungsstarken Laserstrahls, der über die Oberfläche geführt wird. Dabei werden jeweils am Ort der hervorstehenden Zacken und Spitzen 33 Materialverdampfung abgerundete Kuppen 35 und am Ort der schartenartigen Vertiefungen 34 durch Materialverlagerung gerundete Lakunen 36 erzeugt werden. Die so umgeformte Oberflächenstruktur weist eine verbesserte Kompatiblität mit der Struktur des Knochengewebes auf.

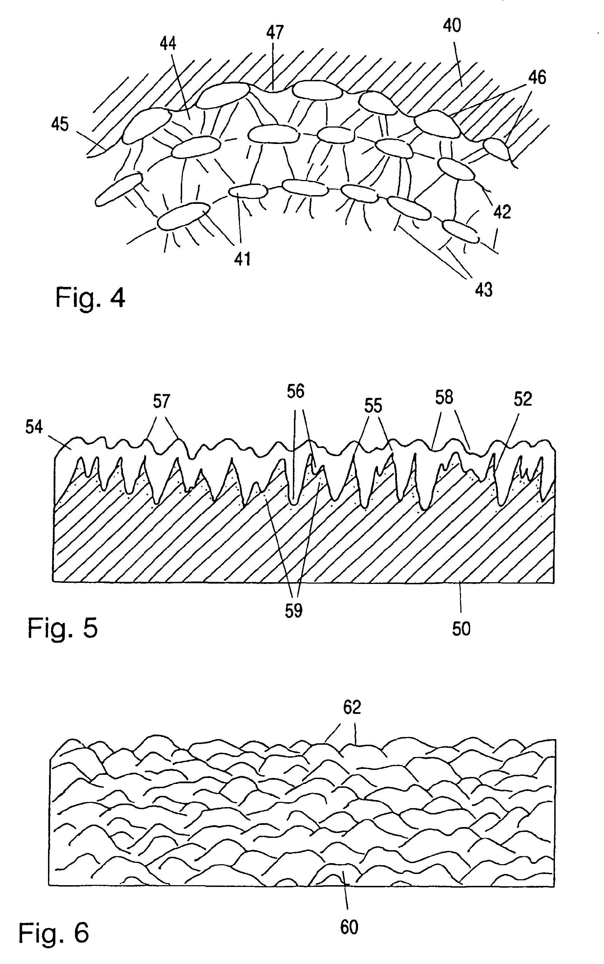

Das sich nach der Operation an das Implantat anlegende Knochengewebe besteht aus Knochenzellen und Zellenfortsätzen, insbesondere aus Osteoblasten, aus diesen entstehende Ostoezyten, welche die Osteozyten miteinander verbinden. Die Osteozyten sind mandelförmig und haben eine Länge von ca. 3-10µm und eine Dicke von ca. 3µm. Die Parameter der Vorbehandlung (Korngröße des Sandstrahlguts) und der Umformung (Leistung des Laserstrahls und Geschwindigkeit der Abtastbewegung) sind so gewählt, dass die Größe der Kuppen 35 und ihre Abstände voneinander sowie die Tiefe der Lakunen 36 an die Größenordnung der Ostoezyten und der diese verbindenden Zellfortsätze angepasst ist. Die sich nach der Operation an der Grenzschicht 45 zum Implantat 40 formierenden Knochenzellen finden so eine an ihre Form und Größe angepasste Struktur vor, die ihre Anlagerung am Implantat begünstigt. Anhand der vereinfachten und schematischen Darstellung von Figur 4 wird dies verdeutlicht. Mandelförmige Osteozyten 41 sind entlang der Knochenlamellen 42 angeordnet und durch Zellfortsätze 43 miteinander verbunden. Zwischen diesen Zellstrukturen befindet sich die unstrukturierte Matrix 44 des Knochengewebes.

Der Vorgang der Anlagerung von Knochenzellen an die Oberfläche des Implantats ist dynamisch. Bei dieser Neuorientierung wandern die Zellen 41 auf der Grenzschicht 45 unter der Wirkung ihrer Zellfortsätze 43. Hierbei lagern sich Zellen 41 in Lakunen 46 der Implantatoberfläche ein. Die gerundeten Kuppen 47 erleichtern die Zellbewegungen und reduzieren die Gefahr, dass es dabei zu Zellschädigungen kommt. Ein ähnlicher Effekt tritt auch an der Oberfläche des eingewachsenen Implantats ein, wenn dieses im Kiefer des Patienten belastet wird. Auch hierbei finden Bewegungen der Knochenzellen im Bereich der Grenzschicht zum Implantat statt. Hierbei werden nicht mehr funktionsfähige Osteozyten unter der Wirkung von Osteoklasten abgebaut, und neu differenzierte Osteoblasten sowie erhalten bleibende Osteozyten und diese umgebende amorphe Knochensubstanz migrieren in frei werdende Lakunen 46. Diese Vorgänge werden durch die gerundeten Strukturen 46, 47 erleichtert oder überhaupt erst ermöglicht.

Zur Abrundung der Zacken und Spitzen 33 und zur Rundung der schartenartigen Vertiefungen 34 kann alternativ zur oben beschriebenen Verwendung von Lasern eine galvanische Behandlung angewendet werden. Dabei wird zum Abtragen der Rauhigkeitsspitzen die Elektrodenanordnung so geschaltet, dass die Implantatoberfläche als Kathode wirkt. Hierbei erfolgt auch ein Abtragen von Teilen der Wandungen der scharfkantige Vertiefungen, so dass diese zu gerundeten Lakunen umgeformt werden.

Die Umformung der vorbehandelten Implantatoberfläche zu einer aus gerundeten Erhöhungen oder Kuppen und gerundeten Lakunen bestehenden Mikrostruktur kann des weiteren durch Aufbringen einer Deckschicht erfolgen. Hierzu wird auf die durch Sandstrahlen und/oder Säureätzen vorbehandelte Oberfläche 52 eines Implantats 50 in einem weiteren Verfahrensschritt wird vorzugsweise durch Sputtern eine Deckschicht 54 aufgebracht, die eine Glättung der Oberfläche des Implantats 50 bewirkt (Figur 5).

Sputtern, auch als Ionensputtern oder Kathodenzerstäubung bezeichnet, ist ein für sich bekanntes Verfahren, das unter anderem zur Herstellung dünner Überzüge auf metallischen oder nichtmetallischen Oberflächen zum Einsatz kommt und das auch bereits zur Beschichtung von Knochenimplantaten vorgeschlagen worden ist. Es wird hierzu auf die Veröffentlichung von Weigand, A.J. et al, "Ion-beam-sputter modifiaction of the surface morphology of biological implants", Journal Vacuum Society Technology, vol. 14, No.1, Jan./Feb. 1977, S. 326-331, hingewiesen. Durch Sputtern hergestellte Metallschichten weisen eine hohe Härte und eine gute und dauerhafte Haftung auf dem beschichteten Substrat auf.

Als Ausgangsmaterial für den Sputterschritt wird vorzugsweise Titan oder eine Titanlegierung verwendet, das in Anwesenheit eines Schutzgases wie z.B. Argon auf die Implantatoberfläche aufgetragen wird. Die Dicke der aufgesputterten Deckschicht 54 ist von der Dauer des Sputtervorganges und der Zahl der Sputterdurchläufe abhängig und liegt zwischen einigen Zehntel Mikrometer und einigen Mikrometern, vorzugsweise zwischen 0,1µm und 2µm und beispielsweise bei 0,5 µm. Je nach Art des Implantats wird die Deckschicht bis zu einer Dicke aufgesputtert, die annähernd der Oberflächenunebenheit des Zellprofils angepasst ist.

Die Deckschicht 54 legt sich über die durch das Sandstrahlen und/oder Säureätzen erzeugten Zacken und Spitzen 55 füllt zwischen diesen Spitzen befindlichen scharfkantigen und schartigen Vertiefungen 56 teilweise aus. Es wird so eine wellige Mikrostruktur auf der Oberfläche des Implantats erzeugt, die die Zacken und Spitzen 55 in abgerundete Kuppen 57 und die scharfkantigen Tiefenareale in abgerundete Kalotten 58 umformt. Figur 6 zeigt ein Beispiel einer durch Sputtern hergestellten Oberfläche 60 mit gerundeten Kuppen 62. Die Deckschicht 54 weist eine Dicke auf, die annähernd der Oberflächenunebenheit des Zellprofils entspricht. Sie kann beispielsweise eine Dicke zwischen 0,1 und 2µm haben.

Die durch die Deckschicht 54 erzeugte Oberflächenstruktur hat die gleichen Vorteile, wie sie oben anhand der Figuren 3 und 4 erläutert wurden. Ein weiterer Effekt besteht darin, dass durch die Deckschicht 54 Strahlgutreste und andere Verunreinigungen 59, die sich beim Sandstrahlen in die Oberfläche 52 einlagern können, abgedeckt und versiegelt werden. Die Verunreinigungen 59 werden damit von der Knochensubstanz isoliert und können weder Entzündungen verursachen noch das Einwachsen des Implantats in anderer Weise stören.

Alternativ zur Aufbringung durch Sputtern kann die Deckschicht 54 kann auch durch einen galvanischen Niederschlag hergestellt werden, der sich über die Rauhigkeitsspitzen der vorbehandelten Oberfläche des Implantats legt und diese abrundet und zugleich die scharfkantigen Vertiefungen teilweise ausfüllt, so dass diese zu gerundeten Lakunen umgeformt werden.

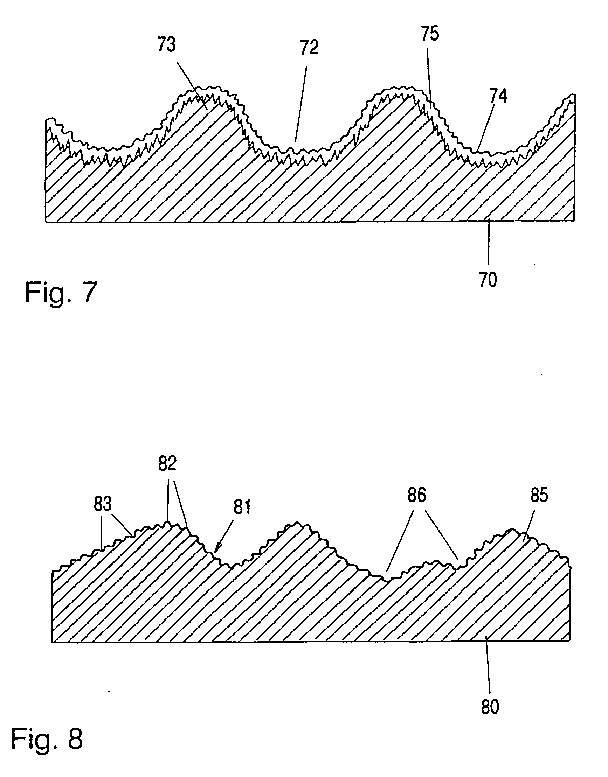

Das Verfahren gemäß der Erfindung kommt vorzugsweise bei Knochenimplantaten zur Anwendung, deren Oberflächen eine Makrostruktur aufweisen. Hierbei handelt es sich um eine Struktur, die geeignet ist, das Implantat belastbar und dauerhaft im Knochen zu verankern wie z.B. Schrauben, selbstschneidende Schrauben, axiale oder diagonale Riffelungen, Nuten oder Rillen. Die Schnittdarstellung von Figur 7 zeigt den Ausschnitt der Oberfläche eines Kieferimplantats 70, dessen Oberfläche eine Makrostruktur in Form rillenartiger Vertiefungen 72 und diese voneinander abgrenzenden Kämmen 73 der in EP-A 1013236 dargestellten Art aufweist. Die Vertiefungen 72 haben eine Größenordnung, die der Größenordnung der Osteone des Knochengewebes entspricht, welche sich an die rillenförmigen Vertiefungen anlagern. Die durch die rillenartigen Vertiefungen 72 und die Kämme 73 gebildete Makrostruktur wird sandgestrahlt und/oder säuregeätzt, so dass über die gesamte Oberfläche der Vertiefungen 72 und Kämme 73 eine einheitliche Rauhigkeit 74 erzeugt wird, die der Mikrostruktur der Oberfläche 12 in Figur 1 entspricht. Über die so behandelte Oberfläche wird eine Deckschicht 75 durch Sputtern oder galvanischen Niederschlag aufgebracht, die die rillenartigen Vertiefungen überdeckt. Es entsteht so eine zellfreundliche Oberfläche, die in hohem Grande biologisch kompatibel ist im Sinne einer wirksamen zellintegration.

In einem weiteren Verfahrensschritt kann der erfindungsgemäß ausgestalteten Mikrostruktur eine Nanostruktur überlagert werden, die in Ihrer Form der beschriebenen Mikrostruktur entspricht und ebenfalls gerundete Kuppen und gerundete Lakunen aufweist. Die Abmessungen der Kuppen, ihre Abstände und die Lakunentiefe der Nanostruktur ist annähernd um eine dezimale Größenordnung kleiner als die der beschriebenen Mikrostruktur. Die Lakunentiefe der Nanostruktur kann im Bereich von 10-500nm, beispielsweise bei 250nm liegen, und der Abstand zwischen den Erhöhungen kann im Bereich von 100-500nm liegen, beispielsweise bei 250nm. Die Figur 8 zeigt bei einem Implantat 80 ein Beispiel einer aus einer Vielzahl von kleinen Kuppen 82 und Lakunen 83 bestehenden Nanostruktur 81, die auf den die Erhebungen 85 und Lakunen 86 der erfindungsgemäßen Mikrostruktur aufgebracht sind. Die Erhebungen 85 und Lakunen 86 entsprechen in ihrer Form und Anordnung den Erhebungen 35 und Lakunen 36 der Mikrostruktur. Die Nanostruktur wird durch einen weiteren Verfahrensschritt auf einer erfindungsgemäß umgeformten Implantatoberfläche hergestellt. Dies geschieht vorzugsweise durch Sputtern, wobei die Parameter des Sputterdurchlaufs zur Herstellung der feinen Struktur entsprechend eingestellt werden. Alternativ kann die Nanostruktur durch Sputtern einen fein fokussierten Laserstrahl hergestellt werden, der entsprechend der herzustellenden Struktur moduliert wird. Die Nanostruktur hat den Effekt, dass die Zellfortsätze in die Lakunen eingreifen und sich dort anlagern können, wodurch ihre Funktion bei der Zellmigration unterstützt wird.

Bei der Herstellung der Mikrostruktur wird die Vorbehandlung durch Sandstrahlen und/oder Säureätzen und die anschließende erfindungsgemäße Umformung, einschließlich der Aufbringung einer Deckschicht, auf diejenigen Teile der Oberfläche des Implantats beschränkt, die mit der Knochensubstanz in Kontakt stehen, nachdem das Implantat eingesetzt worden ist. Hierzu wird eine Maskierung der Oberfläche des Implantats vorgenommen, durch die die zu behandelnden Oberflächenteile von den anderen Oberflächenteilen des Implantats abgegrenzt werden, wobei die anderen Oberflächenteile während der Vorbehandlung und der Umformung abgedeckt werden.

Während die Erfindung anhand einer bevorzugten Ausführungsform beschrieben wurde, können Abwandlungen und andere Ausführungsformen realisiert werden, ohne den durch die Ansprüche definierten Bereich der Erfindung zu verlassen.

## Patentansprüche

1. Knochenimplantat, insbesondere Zahnimplantat, mit einem vorzugsweise aus Titan oder einer Titanlegierung bestehenden Implantatkörper (30), der eine knochenverträgliche Oberfläche (32) besitzt, die eine Makrostruktur zur Anlagerung von Knochensubstanz und eine Mikrostruktur zur Verankerung des Implantats im Zellbereich aufweist, **dadurch gekennzeichnet, dass** über einer durch eine Vorbehandlung mittels Sandstrahlen und/oder Säureätzen aufgerauhten Oberfläche (52) des Implantatkörpers (50) eine aus Titan oder einer Titanlegierung bestehende Deckschicht (54) vorgesehen ist, die vom Sandstrahlen und/oder Säureätzen erzeugte Spitzen und schartenartige Vertiefungen zu einer eine Vielzahl gerundeter Erhöhungen (57) und gerundeter Lakunen (58) aufweisenden Mikrostruktur glättet, und dass die Größe der Erhöhungen und die Tiefe der Lakunen der Größenordnung der Knochenzellen angepasst ist.

2. Implantat nach Anspruch 1, **dadurch gekennzeichnet, dass** die Deckschicht (54) eine durch Sputtern aufgebrachte Schicht ist, deren Dicke wenigstens annähernd der Dicke der Knochenzellen entspricht.

3. Implantat nach Anspruch 1 oder 2, **dadurch gekennzeichnet, dass** die Deckschicht (54) eine Dicke zwischen 0,1 und 2 Mikrometern aufweist.

4. Implantat nach einem der Ansprüche 1 bis 3, **dadurch gekennzeichnet, dass** die Vorbehandlung und die Deckschicht auf Teile der Implantatoberfläche beschränkt sind, die von anderen Teilen der Implantatoberfläche durch deren Maskierung abgegrenzt sind.

5. Knochenimplantat nach Anspruch 1, **dadurch gekennzeichnet, dass** der durch die Deckschicht (54) gebildeten Mikrostruktur (85, 86) eine gleichartige Nanostruktur (81) überlagert ist, deren Erhöhungen (82), ihre Abstände voneinander und die Tiefe der Lakunen (83) annähernd um eine dezimale Größenordnung kleiner sind als die der Mikrostruktur.

6. Knochenimplantat nach Anspruch 5, **dadurch gekennzeichnet, dass** die Lakunentiefe der Nanostruktur (81) im Bereich von 10-500nm und der Abstand zwischen den Erhöhungen (82) im Bereich von 100-500nm liegen.

7. Knochenimplantat nach Anspruch 5, **dadurch gekennzeichnet**, das die Nanostruktur (81) eine durch Sputtern aufgebrachte Schicht ist.

8. Verfahren zur Herstellung eines Knochenimplantats, insbesondere eines Zahnimplantats, mit einem vorzugsweise aus Titan oder einer Titanlegierung bestehenden Implantatkörper (50), der eine biokompatible Oberfläche (52) aufweist, die eine Makrostruktur zur Anlagerung von Knochensubstanz und eine Mikrostruktur zur Verankerung des Implantats im Zellbereich aufweist, **dadurch gekennzeichnet, dass** die Oberfläche (52) durch eine Vorbehandlung mittels Sandstrahlen und/oder Säureätzen aufgerauht wird und dass auf die vorbehandelte Oberfläche (52) des Implantatkörpers (50) eine aus Titan oder einer Titanlegierung bestehende Deckschicht (54) aufgebracht wird, durch die vom Sandstrahlen und/oder Säureätzen der Vorbehandlung erzeugte Spitzen und schartenartige Vertiefungen zu einer eine Vielzahl gerundeter Erhöhungen (Kuppen) (57) und gerundeter Lakunen (58) aufweisenden Mikrostruktur umgeformt werden, wobei die Größe der Erhöhungen und die Tiefe der Lakunen der Größenordnung der Knochenzellen angepasst ist.

9. Verfahren nach Anspruch 8, **dadurch gekennzeichnet, dass** die Deckschicht (54) durch Sputtern aufgebracht wird.

10. Verfahren nach Anspruch 8, **dadurch gekennzeichnet, dass** die Deckschicht (54) durch Elektroplattieren aufgebracht wird.

11. Verfahren nach einem der Ansprüche 8 bis 10, **dadurch gekennzeichnet, dass** die Deckschicht (54) bis zu einer Dicke aufgebracht wird, die annähernd der Dicke der Knochenzellen entspricht

12. Verfahren nach einem der Ansprüche 8 bis 11, **dadurch gekennzeichnet, dass** die Deckschicht (54) in einer Dicke zwischen 0,1 und 2 Mikrometern aufgebracht wird.

13. Verfahren nach einem der Ansprüche 8 bis 12, **dadurch gekennzeichnet, dass** die durch das Sandstrahlen und/oder Säureätzen und das Aufbringen der Deckschicht (54) zu behandelnden Oberflächenteile des Implantats durch Maskierung von anderen Oberflächenteilen des Implantats abgegrenzt und während der Ausführung der Verfahrensschritte abgedeckt bleiben.

14. Verfahren nach einem der Ansprüche 8 bis 13, **dadurch gekennzeichnet, dass** auf die durch die Deckschicht (54) gebildeten Mikrostruktur (85, 86) eine gleichartige Nanostruktur (81) auf eine Makrostruktur (72, 73) der Implantatoberfläche aufgebracht wird, deren Erhöhungen, Abstände und Lakunentiefe annähernd um eine dezimale Größenordnung kleiner ist als die der Mikrostruktur.

15. Verfahren nach Anspruch 14, **dadurch gekennzeichnet, dass** die Lakunentiefe der Nanostruktur (81) im Bereich von 10-500nm und der Abstand zwischen den Erhöhungen (82) im Bereich von 100-500nm liegen.

16. Verfahren nach den Ansprüchen 14 und 15, **dadurch** gekenntzeichnet, dass die Aufbringung der Nanostruktur (81) durch Sputtern erfolgt.

## Claims

1. A bone implant, in particular a dental implant, with an implant body (30) consisting preferably of titanium or a titanium alloy and having a surface (32) that is compatible with bone and having a macrostructure for coming in contact with bone substance and a microstructure for anchoring the implant in the cell area, **characterized in that** a cover layer (54) is arranged on a surface (52) of the implant body (50) which is pretreated by sandblasting and/or acid etching, the cover layer is consists of titanium or a titanium alloy which smooths sharp-edged points and notch-like indentations, generated by the sandblasting and/or acid etching, to a microstructure comprising a plurality of rounded elevations (57) and rounded lacunas (58), and that the size of the elevations and the depth of the lacunas corresponds to the size order of the bone cells.

2. Implant according to Claim 1, **characterized in that** the cover layer (54) is deposited by sputtering and comprises a thickness which corresponds at least approximately to the thickness of the bone cells.

3. Implant according to Claims 1 or 2, **characterized in that** the cover layer (54) has a thickness between 0.1 and 2 micrometers.

4. Implant according to any one of Claims 1 to 3, **characterized in that** the pretreatment and the cover layer are restricted to parts of the implant surface which are delineated from other surface parts of the implant surface by their masking.

5. Bone implant according to Claim 1, **characterized in that** the microstructure (85, 86) formed by the cover layer (54) is superimposed by a similar nanostructure (81) the elevations (82) of the nanostructure, their distances from one another and the depth of the lacunas (83) are smaller than those of the microstructure by approximately one decimal order of magnitude.

6. Bone implant according to Claim 5, **characterized in that** the depth of the lacunas of the nanostructure (81) is in the range of 10-500 nm and the distance between the elevations (82) is in the range of 100-500 nm.

7. Bone implant according to Claim 5, **characterized in that** the nanostructure (81) is a layer deposited by sputtering.

8. A method of producing a bone implant, in particular a dental implant, comprising an implant body (50) which preferably consists of titanium or a titanium alloy and comprises a biocompatible surface (52) having a macrostructure for coming in contact with bone substance and a microstructure for anchoring the implant in the cell area, **characterized in that** the surface (52) is roughened by a pretreatment by means of sandblasting and/or acid etching, and that a cover layer (54), consisting of titanium or a titanium alloy, is deposited on the pretreated surface (52) of the implant body (50), that the sharp-edged points and notch-like indentations, generated by sandblasting and/or acid etching of the pretreatment, are reshaped by the cover layer to a microstructure comprising a plurality of rounded elevations (57) and rounded lacunas (58), and that the size of the elevations and the depth of the lacunas corresponds to the size order of the bone cells.

9. Method according to Claim 8, **characterized in that** the cover layer (54) is deposited by sputtering.

10. Method according to Claim 8, **characterized in that** the cover layer (54) is deposited by electroplating.

11. Method according to one of the Claims 8 to 10, **characterized in that** the cover layer (54) is deposited up to a thickness which corresponds approximately to the thickness of the bone cells.

12. Method according to one of the Claims 8 to 11, **characterized in that** the cover layer (54) is deposited in a thickness between 0.1 and 2 micrometers.

13. Method according to one of the Claims 8 to 12, **characterized in that** the surface parts of the implant treated by sandblasting and/or acid etching and by deposition of the cover layer (54) are delineated from other surface parts of the implant by masking, and the other surface parts remain covered while the method steps are being carried out.

14. Method according to one of the Claims 8 to 13, **characterized in that** the microstructure (85, 86) formed by the cover layer (54) is superimposed by a similar nanostructure (81) deposited on a macrostructure (72, 73) of the implant surface; the elevations of the nanostructure, their distances from one another and the depth of the lacunas are smaller than those of the microstructure by approximately one decimal order of magnitude.

15. Method according to Claims 14, **characterized in that** the depth of the lacunas of the nanostructure (81) is in the range of 10-500 nm and the distance between the elevations (82) is in the range of 100-500 nm.

16. Method according to the Claims 14 and 15, **characterized in that** the nanostructure (81) is deposited by sputtering.

## Revendications

1. Implant osseux, notamment implant dentaire, comportant un corps d'implant (30) réalisé de préférence en titane ou dans un alliage de titane, possédant une surface (32) compatible avec le tissu osseux qui présente une macrostructure permettant l'accumulation de substance osseuse et une microstructure destinée à ancrer l'implant dans la zone des cellules, **caractérisé en ce qu'**il est prévu une couche de finition (54) en titane ou en alliage de titane au-dessus d'une surface (52) brute du corps d'implant (50) piquée lors d'un prétraitement par sablage et/ou décapage à l'acide, que les pointes et creux en forme de brèches produits par sablage et/ou décapage à l'acide sont lissés en une microstructure présentant une multitude d'élévations (57) arrondies et de lacunes (58) arrondies et **en ce que** la dimension des élévations et la profondeur des lacunes sont adaptées à l'ordre de grandeur des cellules osseuses.

2. Implant selon la revendication 1, **caractérisé en ce que** la couche de finition (54) est une couche appliquée par pulvérisation cathodique dont l'épaisseur correspond au moins approximativement à l'épaisseur des cellules osseuses.

3. Implant selon la revendication 1 ou 2, **caractérisé en ce que** la couche de finition (54) présente une épaisseur comprise entre 0,1 et 2 microns.

4. Implant selon l'une des revendications 1 à 3 **caractérisé en ce que** le prétraitement et la couche de finition sont cantonnés à des parties de la surface de l'implant, délimitées par leur masquage par rapport à d'autres parties de la surface de l'implant.

5. Implant osseux selon la revendication 1 **caractérisé en ce que** la microstructure (85,86) formée par la couche de finition (54) est recouverte par une nanostructure (81) de même type, dont les élévations (82), leurs intervalles les unes par rapport aux autres et la profondeur des lacunes (83) sont plus petits d'approximativement un ordre de grandeur décimal par rapport à ceux de la microstructure.

6. Implant osseux selon la revendication 5 **caractérisé en ce que** la profondeur des lacunes de la nanostructure (81) est de l'ordre de 10-500 nm et l'intervalle entre les élévations (82) de l'ordre de 100-500 nm.

7. Implant osseux selon la revendication 5 **caractérisé en ce que** la nanostructure (81) est une couche appliquée par pulvérisation cathodique.

8. Procédé de fabrication d'un implant osseux, notamment d'un implant dentaire, comportant un corps d'implant (50) réalisé de préférence en titane ou en alliage de titane, qui présente une surface (52) biocompatible présentant une macrostructure pour le dépôt de substance osseuse et une microstructure pour ancrer l'implant dans la zone des cellules, **caractérisé en ce que** la surface (52) est piquée par un prétraitement par sablage et/ou décapage à l'acide et **en ce qu'**on applique sur la surface (52) prétraitée du corps d'implant (50) une couche de finition (54) en titane ou en alliage de titane, par laquelle les pointes et les creux en forme de brèches produits par le prétraitement par sablage ou et/ou par décapage à l'acide sont transformés en une microstructure présentant une multitude d'élévations (coupoles) (57) arrondies et de lacunes (58) arrondies, la dimension des élévations et la profondeur des lacunes étant adaptées à l'ordre de grandeur des cellules osseuses.

9. Procédé selon la revendication 8 **caractérisé en ce que** la couche de finition (54) est appliquée par pulvérisation cathodique.

10. Procédé selon la revendication 8 **caractérisé en ce que** la couche de finition (54) est plaquée par électrolyse.

11. Procédé selon l'une des revendications 8 à 10 **caractérisé en ce que** la couche de finition (54) est appliquée jusqu'à une épaisseur qui correspond approximativement à l'épaisseur des cellules osseuses.

12. Procédé selon l'une des revendications 8 à 11, **caractérisé en ce que** la couche de finition (54) est appliquée à une épaisseur comprise entre 0,1 et 2 microns.

13. Procédé selon l'une des revendications 8 à 12 **caractérisé en ce que** les parties de la surface de l'implant traitées par sablage et/ou décapées à l'acide et destinées à recevoir une couche de finition (54) restent recouvertes pendant la réalisation des étapes de traitement et resteront délimitées, par masquage, des autres parties de la surface de l'implant.

14. Procédé selon l'une des revendications 8 à 13, **caractérisé en ce qu'**il est appliqué sur la microstructure (85, 86) formée par la couche de finition (54), une nanostructure (81) de même type sur une macrostructure (72, 73) de la surface de l'implant dont les élévations, intervalles et profondeur des lacunes (83) sont plus petits d'approximativement un ordre de grandeur décimal par rapport à ceux de la microstructure.

15. Procédé selon la revendication 14 **caractérisé en ce que** la profondeur des lacunes de la nanostructure (81) est de l'ordre de 10-500 nm et l'intervalle entre les élévations (82) de l'ordre de 100-500 nm.

16. Procédé selon les revendications 14 et 15 **caractérisé en ce que** l'application de la nanostructure (81) se fait par pulvérisation cathodique.
